(19) Europäisches Patentamt
European Patent Office
Office européen des brevets

(11) Veröffentlichungsnummer: **0 107 846**
**B1**

(12) # EUROPÄISCHE PATENTSCHRIFT

(45) Veröffentlichungstag der Patentschrift: **07.10.87**

(51) Int. Cl.⁴: **A 61 K 7/48**

(21) Anmeldenummer: **83110512.7**

(22) Anmeldetag: **21.10.83**

(54) **Mittel zur Pflege oder Behandlung der menschlichen Haut.**

(30) Priorität: **23.10.82 DE 3239317**

(43) Veröffentlichungstag der Anmeldung:
**09.05.84 Patentblatt 84/19**

(45) Bekanntmachung des Hinweises auf die Patenterteilung:
**07.10.87 Patentblatt 87/41**

(84) Benannte Vertragsstaaten:
**AT CH DE FR GB LI NL**

(56) Entgegenhaltungen:
**DE - A - 2 421 695**
**DE - A - 2 533 140**
**FR - A - 2 422 398**

**CHEMICAL ABSTRACTS, Band 94, Nr. 14, April 1981, Seite 381, Nr. 109088b, Columbus, Ohio, US**
**CHEMICAL ABSTRACTS, Band 88, 1978, Seite 239, Nr. 47065g, Columbus, Ohio, US**
**CHEMICAL ABSTRACTS, Band 56, 1962, Zusammenfassung Nr. 6075g, Columbus, Ohio, US, YOSHIHIRO NITTA u.a.: "Syntheses of 1-deoxy-1-ureido-D-glucuronic acid and related compounds. I. Syntheses of 1-deoxy-1-thioureido-beta-D-gluco pyranuronic acid and its amide"**
**CAS REGISTRY HANDBOOK. Seite 1774R**
**Die Akte enthält technische Angaben, die nach dem Eingang der Anmeldung eingereicht wurden und die nicht in dieser Patentschrift enthalten sind.**

(73) Patentinhaber: **Weber, Gerhard, Prof. Dr., Lempenmühle, D-8602 Mühlhausen (DE)**
Patentinhaber: **Schrader, Karlheinz, Dipl.-Ing., Max-Planck-Strasse 6, D-3450 Holzminden 1 (DE)**

(72) Erfinder: **Weber, Gerhard, Prof. Dr., Lempenmühle, D-8602 Mühlhausen (DE)**
Erfinder: **Schrader, Karlheinz, Dipl.-Ing., Max-Planck-Strasse 6, D-3450 Holzminden 1 (DE)**

(74) Vertreter: **Richter, Bernhard, Dipl.-Ing., Beethovenstrasse 10, D-8500 Nürnberg 20 (DE)**

**EP 0 107 846 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Hautpflege- und Hautschutzmittel mit einem Gehalt an einem Hautglättungsmittel.

Es ist allgemein bekannt, dass zum gesunden Aussehen der menschlichen Haut eine hinreichende Hautglätte zählt, für die hauteigene Substanzen verantwortlich gemacht werden. Werden diese Substanzen der Haut durch äussere Einflüsse, wie häufiges Waschen mit Chemikalien sowie durch witterungsbedingte Einflüsse, entzogen, so tritt ein Wassermangel an der Hornschicht ein und es entsteht das Erscheinungsbild einer schuppigen und rauhen Hautbeschaffenheit aufgrund eines unzureichenden Hautschutzfilms.

Es sind zwar bereits zahlreiche kosmetische Hautschutzmittel, beispielsweise Salicylsäure, zur Entschuppung der Haut bekannt. Diese führt jedoch bekanntlich zu einer verstärkten Hornhautbildung, insbesondere von Handteller und Fusssohle, so dass ihr Dauergebrauch zu nachhaltigen Nebenwirkungen führt.

So sind aus Chemical Abstracts 94: 1 090 886 und Jpn. Kokai 80 153 711 Zusammensetzungen zur kosmetischen Anwendung auf der Haut mit einem Gehalt an Hyaluronsäure bekannt.

Darüber hinaus hat man zur Behandlung von Hauterkrankungen Uronsäure (D-Glucuronsäure) und deren Derivate (wie z.B. D-Glucuronsäuregamma-lacton oder D-Glucuronsäure selbst) zur Behandlung von Hauterkrankungen eingesetzt (vgl. DE-A-2 533 140 und DE-A-2 421 695).

Ferner wird aus Chemical Abstracts 56: 6075 g die Synthese von Harnstoffkondensaten, wie der 1-deoxy-1-thioureido-β-D-glucopyranuronsäure und ihrer Amide beschrieben. Eigenschaften dieser Verbindungen werden nicht erwähnt.

Schliesslich wurden zur Pflege und Behandlung trockener Haut Umsetzungsprodukte aus verschiedenen alpha- und beta-Hydroxysäuren sowie Ketosäuren, wie z.B. der D-Glucuronsäure, und einem Diamin bis zu 8 Kohlenstoffatomen verwendet, ohne jedoch in der Behandlung von schuppenden Hauterscheinungen mit auffallender Rissbildung nachhaltige und sichtbare Erfolge zu erzielen (vgl. FR-PS 2 422 398). Vielmehr ging der Behandlungserfolg mit diesen Umsetzungsprodukten oder deren Estern, Amiden und Salzen der entsprechenden Säuren sowie mit deren Säuren selbst nicht über jenen mit der D-Glucuronsäure allein hinaus.

Diese bekannten Zubereitungen und Verbindungen, wie auch andere handelsübliche Produkte, haben jedoch aufgrund ihrer relativ unzureichenden hautglättenden Wirkung in der Praxis nur eine geringe Bedeutung erlangt. Ebensowenig konnte der Harnstoff, dem eine wasserretinierende Eigenschaft zukommt, den Vorstellungen der Fachwelt gerecht werden.

Der vorliegenden Erfindung lag daher die Aufgabe zugrunde, neue Hautpflegemittel bereitzustellen, die bereits in geringer Wirkstoffkonzentration eine hohe Wirksamkeit als glättendes Hautschutzmittel entfalten. Insbesonders war es Aufgabe der Erfindung, die oftmals als lästig empfundenen Hautschuppen, die der menschlichen Haut ein unansehnliches bis krankhaftes Aussehen verleihen und die in den verschiedensten Erscheinungsformen auftreten, auszuschalten, das heisst, eine «Entschuppung» der Haut vorzunehmen und damit der Haut wieder ein angenehmes Äusseres zu verleihen.

Diese Aufgabe wird gelöst, durch Mittel zur Pflege und Behandlung der menschlichen Haut, gekennzeichnet durch einen Gehalt an G-Glucuronsäureharnstoffkondensat der Formel

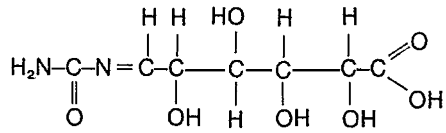

In einer weiteren bevorzugten Ausgestaltung der Erfindung enthalten die Mittel zur Pflege und Behandlung der menschlichen Haut 1–9%, insbesondere 5 Gew.-% D-Glucuronsäureharnstoffkondensat der obigen Formel, bezogen auf die Gesamtzusammensetzung.

Insbesondere werden Mittel bevorzugt, die sich durch folgende Zusammensetzung auszeichnen:

| | |
|---|---|
| Sorbitanmonooleat | 3,0% |
| Paraffinöl perl. | 20,0% |
| Konservierungsmittel | 0,2% |
| Kondensationsprodukt aus Propylenoxid und Ethylenoxid | 3,0% |
| Synthetisches Wachs- von langkettigen Fettsäuren und Fettalkoholen | 5,0% |
| Kondensationsprodukt aus Harnstoff und D-Glucuronsäure 50%ige wässrige Lösung | 10,0% |
| Wasser | ad. 100,0% |

Insbesondere weisen die erfindungsgemässen Mittel die Zusammensetzung auf:

| | |
|---|---|
| Paraffinöl perl. | 15,5% |
| Konservierungsmittel | 0,2% |
| Glycerinmonostearat | 6,0% |
| Stearinsäure | 2,5% |
| Cetylalkohol | 1,0% |
| Walrat | 1,5% |
| 2-Ethylhexylpalmitat | 3,0% |
| Vaselin® | 4,0% |
| Kondensationsprodukt aus Harnstoff und D-Glucuronsäure, 50%ige wässrige Lösung | 10,0% |
| Methylolgruppen enthaltende Imidazolidinylharnstoffverbindung | 0,45% |
| Wasser | ad. 100,0% |

Bei den erfindungsgemässen Mitteln liegt das D-Glucuronsäureharnstoffkondensat in einer W/O-Emulsion eingearbeitet vor.

Es konnte nämlich überraschend festgestellt werden, dass ein Kondensationsprodukt aus D-Glucuronsäure und Harnstoff gegenüber der Anwendung von D-Glucuronsäure bzw. ihrem Natriumsalz in gleicher Konzentration eine weitaus höhere Hautglättung bewirkt. Dies war insofern überraschend, als man in Kenntnis der französischen Patentschrift 2 422 398 davon ausgehen

musste, dass der hautglättende Effekt ausschliesslich dem D-Glucuronsäurerest zugeschrieben werden muss. Aus diesem Grund war bei dem erfindungsgemäss eingesetzten D-Glucuronsäureharnstoffkondensat, das nach Kondensation keinen Glucuronsäurerest mehr aufweist, ein Hautglättungseffekt nicht zu erwarten.

Die Herstellung der D-Glucuronsäure und des Harnstoffes ist ebenso bekannt, wie das erfindungsgemäss eingesetzte D-Glucuronsäureharnstoffkondensat (vgl. z.B. Chemical Abstracts 56: 6075 sowie Methods in Carbohydrate Chemistry, Vol. II, Academic Press Inc. New York and London, 1963, Seite 11 ff).

Das erfindungsgemäss verwendete Kondensat aus Harnstoff und D-Glucuronsäure ist sehr leicht wasserlöslich und unlöslich in organischen Lösungsmitteln. Es zerfällt beim Konzentrieren der wässrigen Lösung sowie beim Kochen der Alkalien oder verdünnten Mineralsäuren in Harnstoff und D-Glucuronsäure.

Dieses erfindungsgemäss verwendete Produkt ist in vorzüglicher Weise geeignet, die Hautglätte in der Haut wieder herzustellen und der Haut dadurch ihre volle Funktionsfähigkeit wiederzugeben.

Das Kondensationsprodukt aus D-Glucuronsäure mit Harnstoff ist auch in gelösten waschaktiven Substanzen, wie Bade-, Duschbad- und hautpflegenden Geschirrspülmitteln lösbar und in dieser Lösung gemäss der Erfindung anwendbar.

Die Erfindung wird anhand der nachfolgend aufgeführten Beispiele und Ergebnisse belegt, ohne den Gegenstand der Erfindung darauf zu beschränken.

Als Anlage 1 wird eine Tabelle mit entsprechenden Modellrezepturen beschrieben, wobei angegeben ist, ob es sich um eine W/O- oder um eine O/W-Emulsion handelt (in der Kosmetik werden bevorzugt O/W-Emulsionen für am Tage anzuwendende Produkte und W/O-Emulsionen für in der Nacht aufzutragende Produkte vorgesehen).

Die Prüfung der Rauhigkeit der menschlichen Haut mit den genannten Emulsionstypen wurde an 10 Probanden zwischen 10 und 65 Jahren durchgeführt, und zwar bei der Modellemulsion vom Typ O/W (Öl-in-Wasser-Emulsion) gegen Aqua dest. (in der Abbildung 1 mit «W» gekennzeichnet). Dabei stellte sich heraus, dass gegenüber dem Leerwert Haut (in der Abbildung 1 mit «H» bezeichnet) die Verbesserung der Hautglätte bei Anwendung des D-Glucuronsäureharnstoffkondensates am grössten war, gefolgt von den Formulierungen unter Verwendung reiner D-Glucuronsäure und dem Einsatz vom Natriumsalz der Glucuronsäure. Der Placebowert war auch hier wieder am schlechtesten.

Die Abbildung 1 zeigt deshalb grafisch die Beeinflussung der Hautrauhigkeit, die sich bei den einzelnen Versuchen laut Anlage 1 mit den obengenannten 10 Probanden im Alter von 10 bis 65 Jahren ergeben hat. Die Rezepturnummern der

Anlage 1

Modellrezepturen

| Rezeptur-Nr. / Rohstoffe | 271/25 W/O % | /26 W/O % | /27 W/O % | /28 O/W % | /29 O/W % | /30 O/W % | /31 W/O % | /32 O/W % |
|---|---|---|---|---|---|---|---|---|
| Sorbitanmonooleat | 3 | 3 | 3 | | | | 3 | |
| Isopropylstearat | 5 | 5 | 5 | 6 | 6 | 6 | 5 | 6 |
| Paraffinöl perl. | 15 | 15 | 15 | 5,5 | 5,5 | 5,5 | 15 | 5,5 |
| Konservierungsmittel | 0,2 | 0,2 | 0,2 | 0,2 | 0,2 | 0,2 | 0,2 | 0,2 |
| Kondensationsprodukt aus Propylenoxid und Ethylenoxid | 3 | 3 | 3 | | | | 3 | |
| Synthetisches Wachs von langkettigen Fettsäuren und Fettalkoholen | 5 | 5 | 5 | | | | 5 | |
| Glycerinmonostearat | | | | 6 | 6 | 6 | | 6 |
| Lanolinalkohol | | | | 2 | 2 | 2 | | 2 |
| Stearinsäure | | | | 2,5 | 2,5 | 2,5 | | 2,5 |
| Cetylalkohol | | | | 1 | 1 | 1 | | 1 |
| Walrat | | | | 1,5 | 1,5 | 1,5 | | 1,5 |
| 2-Ethylhexylpalmitat | | | | 3 | 3 | 3 | | 3 |
| Vaselin® | | | | 4 | 4 | 4 | | 4 |
| Silikonöl | | | | 1 | 1 | 1 | | 1 |
| Kondensationsprodukt aus Harnstoff und D-Glucuronsäure 50 %ig in $H_2O$ | 10 | | | 10 | | | | |
| D-Glucuronsäure | | 5 | | | 5 | | | |
| D-Glucuronsäure Natriumsalz | | | 5 | | | 5 | | |
| Sorbitol | 5 | 5 | 5 | 5 | 5 | 5 | 5 | 5 |
| $H_2O$ | 53,8 | 58,8 | 58,8 | 51,65 | 56,65 | 56,65 | 63,8 | 61,65 |
| Methylolgruppen enthaltende Imidazolidinylharnstoff-Verbindung | | | | 0,45 | 0,45 | 0,45 | | 0,45 |
| Hydroxyethylcellulose | | | | 0,2 | 0,2 | 0,2 | | 0,2 |

Anlage 1 sind jeweils hinzugefügt. Dabei nimmt die Rauhigkeit an der Ordinate von unten nach oben zu.

Unterhalb der Abszisse sind die Versuche in einer W/O-Emulsion und oberhalb der Abszisse die Versuche in einer O/W-Emulsion aufgetragen. Es zeigt sich, dass die Rauhigkeit mit einer W/O-Emulsion am geringsten ist. Zugleich ist ersichtlich, dass die Versuche der Rezepturnummern 25 und 28 innerhalb ihres jeweiligen Systems W/O oder O/W zum jeweils besten Ergebnis führen, das heisst, zu einer innerhalb des Systems geringsten Rauhigkeit führen. Bei den Versuchen 25 und 28 ist der Anteil des Kondensationsproduktes aus Harnstoff und D-Glucuronsäure 50%ig in Wasser gleich 10%, das heisst, auf 100% bezogen gleich 5%.

Als Untersuchungsmethode für die Bestimmung der Hautrauigkeit wurde die Methode nach Padberg zugrunde gelegt (vgl. J. Soc. Cosm. Chem. 20, Seite 719–728, 1969). Dieser Hautoberflächenzustandstest beruht auf der Bestimmung der Haftfähigkeit von Methylenblau auf der Hornschicht. Die Intensität der Anfärbung ist ein Mass für die Rauhigkeit der Hautoberfläche. Die bei den Versuchen aufgetretene hautglättende Wirkung wurde dabei quantitativ erfasst und in Relation zur unbehandelten Haut gesetzt. Das Ergebnis der Versuche wird durch ihre grafische Darstellung in Abbildung 1 verdeutlicht.

**Patentansprüche**

1. Mittel zur Pflege und Behandlung der menschlichen Haut gekennzeichnet durch einen Gehalt an D-Glucuronsäureharnstoffkondensat der Formel

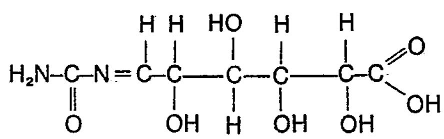

2. Mittel nach Anspruch 1 gekennzeichnet durch einen Gehalt von 1–9%, insbesondere 5 Gew.% D-Glucuronsäureharnstoffkondensat, bezogen auf die Gesamtzusammensetzung.

3. Mittel nach Anspruch 1 gekennzeichnet durch die Zusammensetzung

| | |
|---|---|
| Sorbitanmonooleat | 3,0% |
| Paraffinöl perl. | 20,0% |
| Konservierungsmittel | 0,2% |
| Kondensationsprodukt aus Propylenoxid und Ethylenoxid | 3,0% |
| Synthetisches Wachs von langkettigen Fettsäuren und Fettalkoholen | 5,0% |
| Kondensationsprodukt aus Harnstoff und D-Glucuronsäure 50%ige wässrige Lösung | 10,0% |
| Wasser | ad. 100,0% |

4. Mittel nach Anspruch 1 gekennzeichnet durch die Zusammensetzung

| | |
|---|---|
| Pasraffinöl perl. | 15,5% |
| Konservierungsmittel | 0,2% |
| Glycerinmonostearat | 6,0% |
| Stearinsäure | 2,5% |
| Cetylalkohol | 1,0% |
| Walrat | 1,5% |
| 2-Ethylhexylpalmitat | 3,0% |
| Vaselin® | 4,0% |
| Kondensationsprodukt aus Harnstoff und D-Glucuronsäure 50%ige wässrige Lösung | 10,0% |
| Methylolgruppen enthaltende Imidazolidinylharnstoffverbindung | 0,45% |
| Wasser | ad. 100,00% |

5. Mittel nach einem der Ansprüche 1–4 dadurch gekennzeichnet, dass das D-Glucuronsäureharnstoffkondensat in einer W/O-Emulsion eingearbeitet vorliegt.

**Claims**

1. Preparation for the care or treatment of human skin containing an amount of D-glucuronic acid-urea condensate having the formula

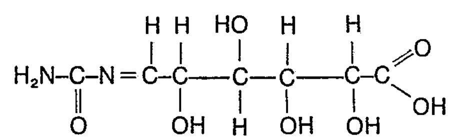

2. Preparation according to claim 1 wherein the D-glucuronic acid-urea condensate is present in an amount of 1–9%, especially 5% by weight with respect to the entire composition.

3. Preparation according to claim 1 having the formula

| | |
|---|---|
| sorbitan monooleate | 3,0% |
| paraffin oil perliquidum | 20,0% |
| preserving agent | 0,2% |
| condensation product of propylene oxide and ethylene oxide | 3,0% |
| synthetic wax composed of long chains of fatty acids and fatty alcohols | 5,0% |
| 50% aqueous solution of the condensation product of urea and D-glucuronic acid | 10,0% |
| water | 100,0% |

4. Preparation of claim 1 composed of

| | |
|---|---|
| paraffin oil perliquidum | 15,5% |
| preserving agent | 0,2% |
| glycerin monostearate | 6,0% |
| stearic acid | 2,5% |
| cetyl alcohol | 1,0% |
| spermaceti | 1,5% |
| 2-ethylhexylpalmitate | 3,0% |
| petrolatum | 4,0% |
| 50% aqueous solution of the condensation product of urea and D-glucuronic acid | 10,0% |

| | |
|---|---|
| compound of imidazolidinyl urea containing methylol groups | 0,45% |
| water | ad. 100,0% |

5. Preparation of one of the claims 1–4 containing the D-glucuronic acid urea condensate integrated in a w/o emulsion.

## Revendications

1. Agent pour le soin et le traitement de la peau humaine, caractérisé par une teneur en produit de condensation acide D-glucuronique/urée de la formule:

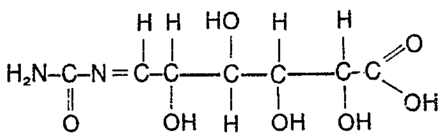

2. Agent selon la revendication 1, caractérisé par une teneur de 1 à 9%, en particulier 5% en poids, en produit de condensation acide D-glucuronique/urée, relativement à la composition d'ensemble.

3. Agent selon la revendication 1, caractérisé par la composition

| | |
|---|---|
| monooléate de sorbitan | 3,0% |
| huile de paraffine fluide | 20,0% |
| conservateur | 0,2% |
| produit de condensation d'oxyde de propylène et d'oxyde d'éthylène | 3,0% |
| cire synthétique d'acides gras et alcools gras à longue chaîne | 5,0% |
| produit de condensation d'urée et d'acide D-glucuronique, solution aqueuse à 50% | 10,0% |
| eau pour faire | 100,0% |

4. Agent selon la revendication 1, caractérisé par la composition

| | |
|---|---|
| huile de paraffine fluide | 15,5% |
| conservateur | 0,2% |
| monostéarate de glycérol | 6,0% |
| acide stéarique | 2,5% |
| alcool cétylique | 1,0% |
| blanc de balein | 1,5% |
| palmitate de 2-éthylhexyl | 3,0% |
| Vaseline (marque déposée) | 4,0% |
| produit de condensation d'urée et d'acide D-glucuronique, solution aqueuse à 50% | 10,0% |
| composé imidazolidinylurée contenant des groupes méthylol | 0,45% |
| eau pour faire | 100,0% |

5. Agent selon l'une des revendications 1 à 4, caractérisé par le fait que le produit de condensation acide D-glucuronique/urée est présent à l'état incorporé à une émulsion eau/huile.

Abbildung 1

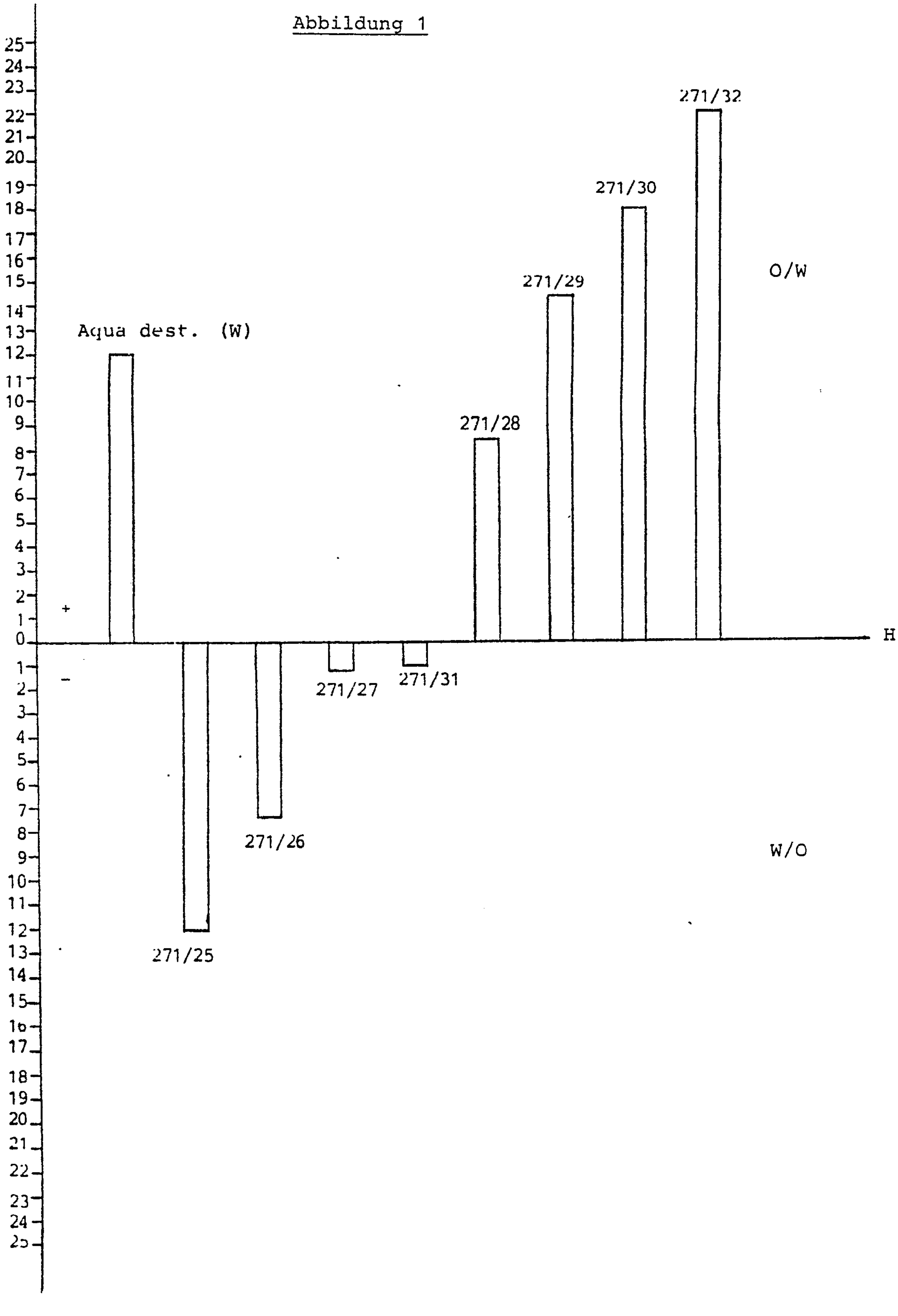

25
24
23
22
21
20
19
18
17
16
15
14
13
12
11
10
9
8
7
6
5
4
3
2
1
0
+
-
1
2
3
4
5
6
7
8
9
10
11
12
13
14
15
16
17
18
19
20
21
22
23
24
25
Aqua dest. (W)
271/25
271/26
271/27
271/31
271/28
271/29
271/30
271/32
O/W
W/O
H